# EUROPÄISCHE PATENTSCHRIFT

(11) **EP 2 872 466 B1**
(45) Veröffentlichungstag und Bekanntmachung des Hinweises auf die Patenterteilung: **01.11.2017**
(21) Anmeldenummer: 13730490.3
(22) Anmeldetag: 18.06.2013
(51) Int. Cl.: C07C 1/24, C07C 45/50, C07C 51/235

(54) **VERFAHREN ZUR HERSTELLUNG VON ISONONANSÄUREN AUS 2-ETHYLHEXANOL**
METHOD FOR PRODUCING ISONONANOIC ACIDS FROM 2-ETHYL HEXANOL
PROCÉDÉ DE PRÉPARATION D'ACIDES ISONANOÏQUES À PARTIR DE 2-ÉTHYLHEXANOL

(30) Priorität: 13.07.2012 DE 102012013969
(43) Veröffentlichungstag der Anmeldung: 20.05.2015
(73) Patentinhaber: OXEA GmbH, 46147 Oberhausen (DE)
(72) Erfinder: FREY, Guido, D., 64560 Riedstadt (DE); EISENACHER, Matthias, 46485 Wesel (DE); KOCKRICK, Kristina, 40505 Düsseldorf (DE); STRUTZ, Heinz, 47445 Moers (DE)
(86) Internationale Anmeldenummer: PCT/EP2013/001798
(87) Internationale Veröffentlichungsnummer: WO 2014/008975

(56) Entgegenhaltungen:
- WO-A1-03/029180
- DE-A1- 2 604 545
- DE-A1- 2 844 638
- DE-A1- 19 908 320

## Beschreibung

Die vorliegende Erfindung betrifft die Herstellung eines Gemisches aus strukturverzweigten C9-Monocarbonsäuren ausgehend von 2-Ethylhexanol durch Dehydratisierung von 2-Ethylhexanol, Hydroformylierung des erhaltenen Octensgemisches zu Isononanal und nachfolgende Oxidation zu der entsprechenden Isononansäure sowie die Herstellung von Vinylisononanoat, dem Glycidylester, Carbonsäureestern, Isononansäurehalogeniden, Isononansäureanhydriden und Isononansäureamiden ausgehend von dieser so hergestellten Isononansäure.

Isononansäure, ein Gemisch aus strukturverzweigten C9-Monocarbonsäuren, ist ein wichtiges Zwischenprodukt in der industriellen organischen Chemie, das zu einer Vielzahl von Folgeprodukten für verschiedenste Anwendungsgebiete verarbeitet wird. Beispielsweise werden ihre Salze als Trocknungsbeschleuniger oder Sikkative für Lacke verwendet und ihre Ester mit Ethylenglykolen dienen als Weichmacher für PVC oder für Polyvinylbutyralfolien sowie als Koaleszenzmittel in wässrigen Dispersionen von Kunststoffen (Weissermel, Arpe, Industrielle Organische Chemie, VCH Verlagsgesellschaft, 3. Auflage, 1988, S. 145; DE 10 2009 048 771 A1). Die Veresterung von Isononansäure mit Polyolen wie Neopentylglykol, Trimethylolpropan, Di-Trimethylolpropan, Pentaerythrit oder Di-Pentaerythrit ergibt Schmiermittelester, die beim Betrieb von Kältemaschinen eingesetzt werden. Isononansäure wird dabei häufig im Gemisch mit anderen C4-C12-Monocarbonsäuren wie 2-Methylbuttersäure, n-Pentansäure, n-Heptansäure, 2-Ethylhexansäure oder n-Octansäure verestert. (EP 1 281 701 A1; EP 1 199 300 A2; EP 0 903 335 A1; WO90/12849 A1; EP 0 475 751 A1).

Des Weiteren wird Isononansäure in den entsprechenden Vinylester überführt, der als Comonomer die Eigenschaften von Polymeren wie Polyvinylacetat, Polyvinylchlorid, Polystyrol oder Polyacrylsäureester modifiziert. Die entsprechenden Copolymere lassen sich zu Anstrichen verarbeiten, die sich durch eine verbesserte Hydrolysebeständigkeit und eine geringere Feuchtigkeitsaufnahme auszeichnen. Vinylester können durch Umsetzung der Isononansäuren mit Acetylen, vorzugsweise in Gegenwart von Zinksalzen bei Temperaturen von 200-230°C hergestellt werden (G. Hübner, Fette, Seifen, Anstrichmittel 68, 290 (1966); Ullmann's Encyclopedia of Industrial Chemistry, 7. Auflage, 2011, Wiley, Band 38, Seiten 107-124; EP 1 057 525 A2) oder durch die sogenannte Transvinylierungsreaktion mit einem Vinylester einer anderen Carbonsäure, häufig Vinylacetat oder Vinylpropionat, in Gegenwart von Übergangsmetallkatalysatoren (Ullmann's Encyclopedia of Industrial Chemistry, 7. Auflage, 2011, Wiley, Band 38, Seiten 107-124; Adelmann, Journal Organic Chemistry, 1949,14, Seiten 1057-1077; DE 199 08 320 A1, EP 0 497 340 A2, WO2011/139360 A1, WO2011/139361 A1).

Als Rohstoff für die industrielle Herstellung von Isononansäure dient der C4-Schnitt aus der Dampfspaltung von Naphtha. Seine Verfügbarkeit im Vergleich zu den C2- und C3-Spaltprodukten kann durch die Bedingungen der Dampfspaltung gesteuert werden und richtet sich nach den Marktgegebenheiten.

Aus den C4-Spaltprodukten wird zunächst 1,3-Butadien durch Extraktion oder durch Selektivhydrierung in n-Butene entfernt. Das erhaltene C4-Raffinat, auch als Raffinat I bezeichnet, enthält überwiegend die ungesättigten Butene Isobuten, 1-Buten und 2-Buten sowie die hydrierten Produkte n-Butan und Isobutan. Aus dem Raffinat I wird im nächsten Schritt Isobuten entfernt und das erhaltene, isobutenfreie C4-Gemisch bezeichnet man als Raffinat II.

Für die Isobutenabtrennung werden in der industriellen Produktion verschiedene Verfahren angewandt, bei denen man die relativ höchste Reaktivität des Isobutens in dem Raffinat I ausnutzt. Bekannt ist die reversible protonenkatalysierte Wasseranlagerung zum tertiär-Butanol oder die Methanolanlagerung zum Methyl-tertiär-butylether. Aus diesen Additionsprodukten kann durch Rückspaltung wieder Isobuten zurückgewonnen werden (Weissermel, Arpe, Industrielle Organische Chemie, VCH Verlagsgesellschaft, 3. Auflage, 1988, Seiten 74-79).

Ebenfalls kann das butadienfreie C4-Raffinat bei erhöhter Temperatur und unter Druck mit einem sauren suspendierten Ionenaustauscher in Kontakt gebracht werden. Isobuten oligomerisiert zu Di-isobuten, Tri-isobuten und in geringem Maße zu höheren Oligomeren. Die Oligomeren werden von den nicht reagierten C4-Verbindungen abgetrennt. Aus dem Oligomerisat kann dann Di-isobuten oder Tri-isobuten destillativ rein gewonnen werden. Durch die Dimerisierung von n-Butenen mit Isobuten wird in geringem Maße Codimer gebildet (Weissermel, Arpe, Industrielle Organische Chemie, VCH Verlagsgesellschaft, 3. Auflage, 1988, S. 77; Hydrocarbon Processing, April 1973, Seiten 171-173).

Di-isobuten, entweder hergestellt durch die Oligomerisierung von durch Rückspaltung erhaltenem reinem Isobuten oder gewonnen im Zuge der Aufarbeitung eines butadienfreien Raffinat I, wird anschließend in ein um ein C-Atom verlängertes C9-Derivat überführt. Technisch betrieben wird die Hydroformylierung oder Oxo-Reaktion, bei der Di-isobuten mit Kohlenmonoxid und Wasserstoff in Gegenwart von Rhodium- oder Kobaltkatalysatoren in den entsprechenden Aldehyd umgewandelt wird. Da Di-isobuten in überwiegendem Maße die Octene 2,4,4-Trimethyl-1-penten und 2,4,4-Trimethyl-2-penten enthält, ergibt die Hydroformylierungsreaktion den C9-Aldehyd 3,5,5-Trimethylhexanal als Hauptbestandteil. Weitere C9-Isomere, die in geringen Mengen zugegen sind, sind 3,4,4- und 3,4,5-Trimethylhexanal sowie 2,5,5-Trimethylhexanal, 4,5,5-Trimethylhexanal und 6,6-Dimethylheptanal. Durch Oxidation dieses Aldehydgemisches erhält man eine technisch verfügbare Isononansäure, die üblicherweise einen Gehalt an 3,5,5-Trimethylhexansäure von etwa 90% aufweist (Ullmanns Encyklopädie der technischen Chemie, 4. Auflage, 1975, Verlag Chemie, Band 9, Seiten 143-145; EP 1 854 778 A1).

Di-isobuten kann ebenfalls nach der sogenannten Hydrocarboxylierung oder Koch-Reaktion mit Kohlenmonoxid und Wasser in Gegenwart von Schwefelsäure in die hochverzweigte Isononansäure 2,2,4,4-Tetramethyl-1-pentansäure überführt werden. Aufgrund der zweifachen Alkylverzweigung an dem der Carboxylgruppe benachbarten Kohlenstoffatom wird diese Isononansäure häufig auch als Neononansäure bezeichnet. (Ullmanns Encyklopädie der technischen Chemie, 4. Auflage, 1975, Verlag Chemie, Band 9, Seiten 143-145).

Auch die nach der Isobutenabtrennung im Raffinat II enthaltenen n-Butene werden technisch zu Butenoligomerisaten umgesetzt, aus denen isomere Octene abgetrennt werden, die über die Hydrocarboxylierung in die entsprechenden Isononansäuren überführt werden (DE 199 08 320 A1; DE 199 06 518 A1). Die Oligomerisierung von n-Butenen wird technisch an sauren Katalysatoren wie Zeolithen oder Phosphorsäure auf Trägem betrieben. Hierbei erhält man Octene, die Dimethylhexene als Hauptprodukt enthalten. Als weitere Verfahren sind der DIMERSOL-Prozess und der OCTOL-Prozess zu nennen. Der DIMERSOL-Prozess arbeitet mit löslichen Nickel-Komplexkatalysatoren und führt zu einem Octengemisch mit einem hohen Anteil an 3- und 5-Methylheptenen neben Dimethylhexenen und n-Octenen. Bei dem OCTOL-Prozess verwendet man geträgerte Nickel-Festbett-Katalysatoren und das erhaltene Octengemisch zeichnet sich durch einen geringen Verzweigungsgrad aus (DE 199 08 320 A1, WO 03/029180 A1, Hydrocarbon Processing, February 1986, Seiten 31-33). Nach DE 199 08 320 A1 werden die jeweiligen, unterschiedlich verzweigten Octengemische über die Hydrocarboxylierung in die entsprechenden Isononansäuren überführt, die anschließend in die entsprechenden Vinylester umgewandelt werden. Vinylester aus Isononansäuren, die auf einem Octengemisch aus dem OCTOL-Prozess basieren, eignen sich als weichmachendes Comonomer.

Vor dem Hintergrund, dass die Verfügbarkeit an Octenen basierend auf dem C4-Schnitt aus der Naphthaspaltung beschränkt ist und von den lokalen Standortbedingungen abhängt, ist es wünschenswert, weitere Octenquellen auf Basis preiswert verfügbarer Großprodukte zu erschließen, die auf einfache Weise zu verschiedenen Standorten transportiert werden können. 2-Ethylhexanol steht als industrielles Großprodukt preiswert zur Verfügung, das ohne Probleme weitläufig vertrieben werden kann. 2-Ethylhexanol wird bekanntermaßen durch Hydroformylierung oder Oxo-Reaktion von Propylen zu n-Butyraldehyd mit nachfolgender alkalisch katalysierter Aldolkondensation zum 2-Ethylhexenal und anschließender Vollhydrierung zum 2-Ethylhexanol großtechnisch hergestellt (Ullmann's Encyclopedia of Industrial Chemistry, 7. Auflage, 2011, Wiley, Band 13, Seiten 579-584).

Auf die Verwendung von 2-Ethylhexanol zur Herstellung eines Octengemisches, das über die Dehydratisierung, Hydroformylierung und Hydrierung zu einem Isononanolgemisch verarbeitet wird, geht WO 03/029180 A1 kurz ein. Dabei steht die Einstellung der Viskosität der isomeren Phthalsäure-dialkylester im Mittelpunkt, die durch Veresterung von isomeren Nonanolen mit Phthalsäure oder Phthalsäureanhydrid erhalten werden. Hinweise, die Dehydratisierungsprodukte von 2-Ethylhexanol in Isononansäure zu überführen, werden nicht gegeben.
Die Nutzung von 2-Ethylhexanol als Octenquelle ermöglicht die Bereitstellung von Isononansäure auf Basis von Propylen und mindert die Abhängigkeit von der Octenverfügbarkeit auf Butenbasis.

Die vorliegende Erfindung besteht daher in einem Verfahren zur Herstellung eines Gemisches aus strukturverzweigten C9-Monocarbonsäuren ausgehend von 2-Ethylhexanol. Das Verfahren ist dadurch gekennzeichnet, dass man
(a) 2-Ethylhexanol in Gegenwart eines Katalysators zu einem Octengemisch dehydratisiert;
(b) das nach Schritt a) erhaltene Octengemisch in Gegenwart einer Übergangsmetallverbindung der Gruppe VIII des Periodensystems der Elemente mit Kohlenmonoxid und Wasserstoff zu Isononanal umsetzt; und
(c) das nach Schritt b) erhaltene Isononanal zu einem Gemisch aus strukturverzweigten C9-Monocarbonsäuren oxidiert.

Die Dehydratisierung von 2-Ethylhexanol kann sowohl in der Flüssigphase als auch in der Gasphase an einem dafür geeigneten Katalysator durchgeführt werden. Bevorzugt erfolgt die Dehydratisierung in der Gasphase bei Temperaturen im Bereich von 200 bis 450°C, vorzugsweise von 250 bis 380°C unter Verwendung fachüblicher Reaktoren in Gegenwart heterogener Katalysatoren mit dehydratisierenden Eigenschaften wie Aluminiumoxid in seinen verschiedenen Modifikationen, Nickel niedergeschlagen auf Aluminiumoxid, oder Phosphorsäure niedergeschlagen auf Siliziumdioxid oder Aluminiumoxid. Solche zur Dehydratisierung geeignete Heterogenkatalysatoren sind aus dem Stand der Technik her bekannt (GB 313426, US 2468764, US 2919973) und stehen beispielsweise als AI3996 der Firma BASF SE kommerziell zur Verfügung. US 2919973 behandelt die Dehydratisierung von 2-Ethylhexanol an einem heterogenen Aluminiumoxidkatalysator bei Temperaturen um 350°C und bei einer Katalysatorbelastung von 2,4 bis 2,8 Liter 2-Ethylhexanol pro Liter Katalysator und Stunde. Der Stand der Technik gibt jedoch keine Auskunft über die Isomerenverteilung in dem erhaltenen Octengemisch.

Der in dem erfindungsgemäßen Verfahren für die Dehydratisierung von 2-Ethylhexanol eingesetzte Reaktor kann neben der Katalysatorschüttung noch weitere Füllkörper oder Einbauten enthalten, beispielsweise Raschigringe, Sättel, Pallringe, Filterplatten oder Kolonnenböden. Verwendet man Füllkörper, dann werden sie vorzugsweise oberhalb der Katalysatorschüttung angebracht, um das Totvolumen zu verringern. Wird in der Flüssigphase dehydratisiert, kann auf Rührvorrichtungen, Einbauten und Füllkörper verzichtet werden, so dass in dem Reaktionsgefäß nur der Dehydratisierungskatalysator anwesend ist. In der bevorzugten Arbeitsweise wird 2-Ethylhexanol in einem vorgeschalteten Verdampfer erhitzt und gasförmig über die Katalysatorschüttung geführt, gegebenenfalls unter Verwendung eines inerten Trägergases wie Stickstoff, Kohlendioxid oder Edelgase. Die Belastung V/Vh des heterogenen Katalysators kann über einen weiten Bereich variieren und beträgt im Allgemeinen von 0,2 bis 3,5 Liter 2-Ethylhexanol pro Liter Katalysator und Stunde. Das der Dehydratisierungszone entnommene Reaktionsgemisch wird anschließend kondensiert. Durch das abgespaltene Wasser fällt eine wässrige Phase an, die von der organischen Olefinphase durch einfache Phasentrennung separiert wird. Bei dem erhaltenen Octen handelt es sich um ein Gemisch strukturisomerer Octene mit den einfach verzweigten Octenen 2-Ethyl-1-hexen sowie cis/trans 3-Methyl-3-hepten und cis/trans 3-Methyl-2-hepten als Hauptkomponenten. Nennenswerte Mengen an Di-C8-Ethern werden nicht gebildet.

Das nach Entfernen des Spaltwassers vorliegende Octen wird anschließend ohne weitere Reinigung oder zweckmäßigerweise nach destillativer Aufreinigung für die Umsetzung mit Kohlenmonoxid und Wasserstoff in der Hydroformylierungsreaktion oder Oxo-Reaktion verwendet. Die eingesetzte Mischung aus Kohlenmonoxid und Wasserstoff bezeichnet man auch als Synthesegas. Man führt die Hydroformylierungsreaktion in einem homogenen Reaktionssystem durch. Der Begriff homogenes Reaktionssystem steht für eine im Wesentlichen aus Lösungsmittel, falls zugesetzt, Katalysator, olefinisch ungesättigter Verbindung und Reaktionsprodukt zusammengesetzte homogene Lösung. Als besonders wirksame Lösungsmittel haben sich die höher siedenden Kondensationsverbindungen der herzustellenden Aldehyde, insbesondere die Trimeren der herzustellenden Aldehyde, erwiesen, die als Nebenprodukte bei der Hydroformylierung anfallen, sowie ihre Mischungen mit dem herzustellenden Isononanal, so dass ein weiterer Lösungsmittelzusatz nicht unbedingt erforderlich ist. In einigen Fällen kann sich jedoch ein Lösungsmittelzusatz als zweckmäßig erweisen. Als Lösungsmittel werden organische Verbindungen eingesetzt, in denen Ausgangsmaterial, Reaktionsprodukt und Katalysator löslich sind. Beispiele für solche Verbindungen sind aromatische Kohlenwasserstoffe, wie Benzol und Toluol oder die isomeren Xylole und Mesitylen. Andere gebräuchliche Lösungsmittel sind Paraffinöl, Cyclohexan, n-Hexan, n-Heptan oder n-Octan, Ether wie Tetrahydrofuran, Ketone oder Texanol® der Firma Eastman. Der Anteil des Lösungsmittels im Reaktionsmedium kann über einen weiten Bereich variiert werden und beträgt üblicherweise zwischen 20 und 90 Gew.-%, vorzugsweise 50 bis 80 Gew.-%, bezogen auf das Reaktionsgemisch. Die Hydroformylierung des Octens kann aber auch ohne Lösungsmittelzusatz erfolgen.

Die Hydroformylierungsreaktion wird typischerweise in homogener organischer Phase in Gegenwart mindestens einer Übergangsmetallverbindung der Gruppe VIII des Periodensystems der Elemente durchgeführt. Die Umsetzung kann sowohl in Gegenwart sowie in Abwesenheit von komplexbildenden Organoelementverbindungen, die als Komplexliganden wirken, durchgeführt werden.

Wird die Hydroformylierungsreaktion in Gegenwart von Komplexliganden durchgeführt, eignet sich die Verwendung von Organophosphorverbindungen als Organoelementverbindungen. Derartige Komplexverbindungen und ihre Herstellung sind bekannt (US 3 527 809 A, US 4 148 830 A, US 4 247 486 A, US 4 283 562 A). Sie können als einheitliche Komplexverbindungen eingesetzt werden. Die Übergangsmetallkonzentration im Reaktionsmedium erstreckt sich über einen breiten Bereich von etwa 1 bis etwa 1000 Gew.-ppm und beträgt vorzugsweise 10 bis 700 Gew.-ppm und insbesondere 25 bis 500 Gew.-ppm, jeweils bezogen auf das homogene Reaktionsgemisch. Als Katalysator kann die stöchiometrisch zusammengesetzte Übergangsmetall-Komplexverbindung Anwendung finden. Es hat sich jedoch als zweckmäßig erwiesen, die Hydroformylierung in Gegenwart eines Katalysatorsystems aus Übergangsmetall-Komplexverbindung und freiem Komplexliganden durchzuführen, der mit dem Übergangsmetall keine Komplexverbindung mehr eingeht. Der freie Komplexligand kann der Gleiche sein wie in der Übergangsmetall-Komplexverbindung, es können aber auch von diesem verschiedene Komplexliganden eingesetzt werden. Zu den bevorzugten Komplexliganden zählen Triarylphosphine wie Triphenylphosphin, Trialkylphosphine wie Tri(cyclohexyl)phosphin, Alkylphenylphosphine, organische Phosphite oder Diphosphite. Das molare Verhältnis von Übergangsmetall zu Komplexligand beträgt im Allgemeinen 1:1 bis 1:1000, es kann aber auch noch höher liegen. Bevorzugt setzt man das Übergangsmetall und den Komplexliganden in einem molaren Verhältnis von 1:3 bis 1:500 und insbesondere von 1:50 bis 1:300 ein.

Die Hydroformylierungsreaktion in Gegenwart von Komplexliganden bezeichnet man häufig auch als modifizierte Variante, die üblicherweise bei Temperaturen von 50 bis 180°C, vorzugsweise von 100 bis 160°C und Gesamtdrücken von 0,2 bis 30 MPa, vorzugsweise von 1 bis 20 MPa durchgeführt wird.

Die Hydroformylierungsreaktion kann ebenfalls in Abwesenheit von Komplexliganden nach der unmodifizierten Variante durchgeführt werden. Solche, beispielsweise nicht mit Phosphinen oder Phosphiten modifizierte Übergangsmetallkatalysatoren und ihre Eignung als Katalysator zur Hydroformylierung sind aus der Literatur her bekannt und sie werden als unmodifizierte Übergangsmetallkatalysatoren bezeichnet. Es wird in der Fachliteratur angenommen, dass die Übergangsmetallverbindung HM(CO)₄ die katalytisch aktive Übergangsmetallspezies bei der unmodifizierten Übergangsmetallkatalyse ist, obgleich dies aufgrund der vielen in der Reaktionszone nebeneinander ablaufenden Chemismen nicht eindeutig bewiesen ist.

Vorzugsweise verwendet man als Übergangsmetalle der Gruppe VIII des Periodensystems der Elemente Kobalt, Rhodium, Iridium, Nickel, Palladium, Platin, Eisen oder Ruthenium und insbesondere Kobalt oder Rhodium. Der modifizierte oder unmodifizierte Übergangsmetallkatalysator bildet sich unter den Bedingungen der Hydroformylierungsreaktion aus den eingesetzten Übergangsmetallverbindungen, wie deren Salzen, wie Chloriden, Nitraten, Sulfaten, Acetaten, Pentanoaten, 2-Ethylhexanoaten oder Isononanoaten, deren Chalkogeniden, wie Oxiden oder Sulfiden, deren Carbonylverbindungen, wie M₂(CO)₈, M₄(CO)₁₂, M₆(CO)₁₆, M₂(CO)₉, M₃(CO)₁₂, deren Organoübergangsmetallverbindungen, wie Carbonylacetylacetonaten oder Cyclooctadienylacetaten oder -chloriden, in Gegenwart von Kohlenmonoxid/Wasserstoffgemischen. Dabei kann die Übergangsmetallverbindung als Feststoff oder zweckmäßigerweise in Lösung eingesetzt werden. Als Übergangsmetallverbindung, die als Katalysatorvorstufe verwendet wird, eignet sich insbesondere Rhodiumisononanoat, Rhodiumacetat, Rhodium-2-ethylhexanoat oder Kobaltisononanoat, Kobaltacetat oder Kobalt-2-ethyl-hexanoat, oder Co₂(CO)₈, Co₄(CO)₁₂, Rh₂(CO)₈, Rh₄(CO)₁₂ oder Rh₆(CO)₁₆ oder Cyclopentadienylrhodiumverbindungen, Rhodiumacetylacetonat oder Rhodiumdicarbonylacetylacetonat. Bevorzugt werden Rhodiumoxid und insbesondere Rhodiumacetat, Rhodium-2-ethylhexanoat und Rhodiumisononanoat eingesetzt.

Es ist aber auch möglich, den Übergangsmetallkatalysator in einer Vorcarbonylierungsstufe zunächst zu präformieren und ihn anschließend der eigentlichen Hydroformylierungsstufe zuzuführen. Die Bedingungen der Präformierung entsprechen dabei im Allgemeinen den Hydroformylierungsbedingungen.

Da im Allgemeinen die Verwendung von nicht mit Komplexliganden modifizierten Übergangsmetallkatalysatoren einen geringeren Übergangsmetallgehalt erfordert, arbeitet man im Allgemeinen mit einer Übergangsmetallmenge von 1 bis 100 ppm, vorzugsweise 2 bis 30 ppm, bezogen auf das eingesetzte Octen. Ganz besonders wird Rhodium oder Kobalt in einer Menge von 2 bis 30 ppm, vorzugsweise von 5 bis 10 ppm, jeweils bezogen auf das eingesetzte Octen, verwendet.

Bei der Umsetzung des Octens mit Wasserstoff und Kohlenmonoxid zu Isononanal nach der unmodifizierten Variante arbeitet man zweckmäßigerweise bei höheren Drücken im Bereich von 5 bis 70 MPa, vorzugsweise von 5 bis 60 MPa und insbesondere von 10 bis 30 MPa. Geeignete Reaktionstemperaturen bewegen sich im Bereich von 50 bis 180°C, bevorzugt von 50 bis 150°C und insbesondere 100 bis 150°C.

Die Zusammensetzung des Synthesegases, d. h. die Anteile von Kohlenmonoxid und Wasserstoff im Gasgemisch, kann in weiten Grenzen variiert werden. Im Allgemeinen setzt man Gemische ein, in denen das Molverhältnis von Kohlenmonoxid zu Wasserstoff 5 : 1 bis 1 : 5 beträgt. Üblicherweise ist dieses Verhältnis 1 : 1 oder weicht von diesem Wert nur wenig ab. Die olefinische Verbindung kann als solche oder in Lösung der Reaktionszone zugeführt werden. Geeignete Lösungsmittel sind Ketone wie Aceton, Methylethylketon, Acetophenon, niedrigere aliphatische Nitrile wie Acetonitril, Propionitril oder Benzonitril, Dimethylformamid, lineare oder verzweigte gesättigte aliphatische Monohydroxyverbindungen wie Methanol, Ethanol, Propanol und Isopropanol, aromatische Kohlenwasserstoffe wie Benzol oder Toluol und gesättigte cycloaliphatische Kohlenwasserstoffe wie Cyclopentan oder Cyclohexan.

Die Hydroformylierungsstufe kann sowohl diskontinuierlich als auch kontinuierlich durchgeführt werden. Die Gewinnung der gewünschten Aldehyde aus dem rohen Hydroformylierungsprodukt erfolgt nach konventionellen Verfahren, beispielsweise durch Destillation. Isononanal und weitere flüchtige Komponenten werden als Kopfprodukte abgezogen und bei Bedarf einer weiteren Feinreinigung unterzogen.

Die eingesetzten Übergangsmetallmengen fallen im Destillationsrückstand an und werden gegebenenfalls nach Zusatz von frischer Übergangsmetallverbindung und Entnahme eines Teils der im Verlauf der Reaktion gebildeten Aldehydkondensationsprodukte in die Reaktionszone zurückgeführt.

Das erhaltene Gemisch isomerer Isononanale wird aufgereinigt, zweckmäßigerweise durch Destillation, und anschließend durch Oxidation in die entsprechende Isononansäure überführt, vorzugsweise durch die Oxidation in der Flüssigphase, obwohl andere Verfahrensausgestaltungen wie die Oxidation in der Gasphase nicht ausgeschlossen sind. Als Oxidationsmittel eignen sich übliche, zur Oxidation von aliphatischen Aldehyden geeignete Verbindungen wie Sauerstoff, sauerstoffenthaltende Gasgemische, Ozon, ozonhaltige Gasgemische, Peroxide, Persäuren, Metallsalze von Persäuren oder Übergangsmetalle in hohen Oxidationsstufen, beispielsweise Kaliumpermanganat oder Braunstein. Aufgrund der guten Verfügbarkeit verwendet man als Oxidationsmittel zweckmäßig molekularen Sauerstoff oder Gasgemische, die molekularen Sauerstoff enthalten. Weitere Bestandteile derartiger Gasgemische sind inerte Gase, z. B. Stickstoff, Edelgase und Kohlendioxid. Der Anteil der inerten Bestandteile des sauerstoffenthaltenden Gasgemisches beträgt bis zu 90 Vol-%, insbesondere 30 bis 80 Vol-%. Die bevorzugten Oxidationsmittel sind Sauerstoff oder Luft.

Die Oxidation kann entweder unter Katalysatorzusatz oder in Abwesenheit von Katalysatoren durchgeführt werden. Als Katalysatoren eignen sich Übergangsmetalle oder Verbindungen von Übergangsmetallen, die in geringen Mengen wie beispielsweise von 0,1 bis 5 ppm, berechnet als Übergangsmetall und bezogen auf eingesetzten Aldehyd, zugesetzt werden können wie Titan, Vanadium, Chrom, Molybdän, Mangan, Eisen, Kobalt, Nickel, Ruthenium, Rhodium, Palladium oder Kupfer. Eine solche Verfahrensführung wird beispielsweise in DE 100 10 771 C1 oder DE 26 04 545 A1 beschrieben.

Ebenfalls kann die Umsetzung in Anwesenheit von Alkali- oder Erdalkalimetallsalzen schwacher Säuren durchgeführt werden. Insbesondere bei der Oxidation von α-verzweigten Aldehyden, bei denen das dem Carbonylkohlenstoffatom benachbarte Kohlenstoffatom die Verzweigung trägt, empfiehlt der Stand der Technik die Anwesenheit von geringen Mengen an Alkalimetallcarboxylaten zur Selektivitätsverbesserung (DE 950 007, DE 100 10 771 C1). Auch kann eine Kombination von Alkali- oder Erdalkalimetallcarboxylaten mit Übergangsmetallverbindungen, wie in EP 1 854 778 A1 behandelt, verwendet werden.

Bei der Oxidation des Isononanals, das nach dem erfindungsgemäßen Verfahren ausgehend von 2-Ethylhexanol über die Dehydratisierung und Hydroformylierung des entsprechenden Octens hergestellt wird, ist die Anwesenheit von Alkali- oder Erdalkalimetallcarboxylaten empfehlenswert, im Allgemeinen in einer Menge von 1 bis 30 mmol, vorzugsweise von 1 bis 15 mmol und insbesondere von 1 bis 8 mmol, je Mol Aldehyd, berechnet als Alkali- oder Erdalkalimetall.

Es ist nicht erforderlich, die Alkali- oder Erdalkalimetallcarboxylate als einheitliche Verbindung einzusetzen. Es ist ebenfalls möglich, Gemische dieser Verbindungen zu verwenden, wobei man jedoch zweckmäßigerweise Isononanoate verwendet. Vorzugsweise setzt man jedoch einheitliche Verbindungen ein, beispielsweise Lithium-, Kalium-, Natrium-, Calcium- oder Bariumisononanoat.

Im Allgemeinen stellt man eine Alkali- oder Erdalkalimetallisononanoate enthaltende Lösung durch Neutralisation einer wässrigen, die Alkali- oder Erdalkalimetallverbindung enthaltenden Lösung mit einem Überschuss an Isononansäure her und setzt diese Lösung dem zu oxidierenden Isononanal zu. Als Alkali- oder Erdalkalimetallverbindungen eignen sich besonders die Hydroxide, Carbonate oder Hydrogencarbonate.

Es ist aber auch möglich, die Alkali- oder Erdalkalimetallisononanoate im Reaktionsgemisch zu erzeugen, indem man Alkali- oder Erdalkalimetallverbindungen zusetzt, die unter den Reaktionsbedingungen in die Isononanoate überführt werden. Beispielsweise lassen sich Alkali- oder Erdalkalimetallhydroxide, -carbonate, -hydrogencarbonate oder -oxide in der Oxidationsstufe einsetzen. Ihr Zusatz kann entweder in fester Form oder als wässrige Lösung erfolgen.

Die Umsetzung mit dem Oxidationsmittel, vorzugsweise mit Sauerstoff oder sauerstoffenthaltenden Gasen, wird in einem Temperaturbereich von 20 bis 100°C durchgeführt. Vorzugsweise arbeitet man zwischen 20 und 80°C, insbesondere zwischen 40 und 80°C. Die Temperaturführung, konstante oder variable Temperatur, kann den individuellen Erfordernissen des Ausgangsmaterials und den Reaktionsgegebenheiten angepasst werden.

Die Umsetzung der Reaktionspartner erfolgt bevorzugt bei Atmosphärendruck. Die Anwendung erhöhten Drucks ist jedoch nicht ausgeschlossen. Üblicherweise arbeitet man in einem Bereich von Atmosphärendruck bis 1,5 MPa, vorzugsweise bei Atmosphärendruck bis 0,8 MPa.

Die zur Umwandlung des Isononanals in die entsprechende Isononansäure erforderliche Reaktionszeit hängt unter anderem ab von der Reaktionstemperatur und dem Mengenverhältnis der Reaktanten zueinander. Normalerweise beträgt sie 30 Minuten bis 20 Stunden, insbesondere 2 bis 8 Stunden.

Isononanal kann als solches oder gelöst in einem, unter den Reaktionsbedingungen inerten, Lösungsmittel eingesetzt werden. Beispiele für geeignete Lösungsmittel sind Ketone wie Aceton, Ester, z. B. Ethylacetat, Kohlenwasserstoffe, z. B. Toluol und Nitrokohlenwasserstoffe wie Nitrobenzol. Die Konzentration des Aldehyds wird durch seine Löslichkeit in dem Lösungsmittel begrenzt.

Der Oxidationsschritt kann absatzweise oder kontinuierlich durchgeführt werden. Eine Rückführung nicht umgesetzter Reaktionsteilnehmer ist in beiden Fällen möglich.

Bei der erhaltenen Isononansäure ausgehend von 2-Ethylhexanol handelt es sich um ein Gemisch stellungsisomerer aliphatischer C9-Monocarbonsäuren mit in α-Position unverzweigten und einfachverzweigten Isononansäuren als Hauptkomponenten.

Nach der gaschromatographischen Analyse gemäß DIN 51405 (FI.-%) liegen als Hauptkomponenten 4-Methyloctansäure, 6-Methyloctansäure, 2,5-Dimethylheptansäure, 2,3-Dimethylheptansäure, 3-Ethylheptansäure, 2-Ethylheptansäure und 2-Ethyl-4-methylhexansäure vor sowie geringe Mengen an 2-Propyl-3-methyl-pentansäure und 2-Methyloctansäure. Geringe Mengen an n-Nonansäure sind ebenfalls zugegen.
Die nach dem erfindungsgemäßen Verfahren hergestellte Isononansäure ist dadurch charakterisiert, dass die Hauptkomponenten 4-Methyloctansäure, 6-Methyloctansäure, 2,5-Dimethylheptansäure, 2,3-Dimethylheptansäure, 3-Ethylheptansäure, 2-Ethylheptansäure und 2-Ethyl-4-methylhexansäure in Summe mindestens 80 mol-%, bezogen auf den Gesamtgehalt an stellungsisomeren aliphatischen C9-Monocarbonsäuren, ausmachen.

Aus dem nach der Oxidation anfallenden Rohsäuregemisch wird mittels Destillation unter üblichen Bedingungen die reine Isononansäure gewonnen. Der die Alkali- oder Erdalkalimetallisononanoate und gegebenenfalls Übergangsmetalle enthaltende Destillationsrückstand wird abgetrennt und kann dem Einsatzaldehyd, gegebenenfalls nach Zugabe von frischen Alkali- oder Erdalkalimetallisononanoaten oder Alkali- oder Erdalkalimetallverbindungen, die unter den Reaktionsbedingungen in die Isononanoate übergehen, sowie gegebenenfalls von frischen Übergangsmetallverbindungen wieder zugeführt werden.

Nach einer bewährten Ausführungsform des erfindungsgemäßen Verfahrens legt man Isononanal in einem geeigneten Reaktor, z. B. in einem mit einem Anströmboden versehenen Rohrreaktor, der gegebenenfalls noch Füllkörper enthält, vor und leitet den Sauerstoff oder das sauerstoffenthaltende Gasgemisch von unten durch den Aldehyd.

Entsprechend einer weiteren Ausführungsform verwendet man als Reaktor einen Rieselturm, der Füllkörper enthält. Über die Füllung lässt man den Aldehyd herabrieseln und leitet in den Turm gleichzeitig im Gleich- oder Gegenstrom Sauerstoff oder ein sauerstoffenthaltendes Gasgemisch ein.

Die nach dem erfindungsgemäßen Verfahren hergestellte Isononansäure kann beispielsweise nach an sich bekannten Verfahren zur Herstellung von Derivaten wie den Vinylester, Carbonsäureestern, Isononansäureanhydriden, Isononansäurehalogeniden oder Isononansäureamiden verwendet werden. Der Vinylester wird beispielsweise hergestellt durch Umsetzung der Isononansäure mit Acetylen, vorzugsweise in Gegenwart von Zinksalzen bei Temperaturen von 200-230°C (G. Hübner, Fette, Seifen, Anstrichmittel 68, 290 (1966), Ullmann's Encyclopedia of Industrial Chemistry, 7. Auflage, 2011, Wiley, Band 38, Seiten 107-124) oder durch die sogenannte Transvinylierungsreaktion 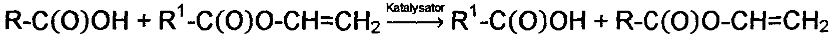 wobei R gleich C8 ist und R¹ häufig Methyl oder Ethyl bedeutet, so dass als Transvinylierungsreagenz beispielsweise Vinylacetat oder Vinylpropionat verwendet wird (Ullmann's Encyclopedia of Industrial Chemistry, 7. Auflage, 2011, Wiley, Band 38, Seiten 107-124). Um das chemische Gleichgewicht in Richtung des gewünschten Vinylesters zu drängen, verwendet man häufig einen Überschuss an dem Transvinylierungsreagenz R¹-C(O)-CH=CH₂ und entfernt gleichzeitig die gebildete Carbonsäure aus dem Reaktionsgemisch. Als Transvinylierungskatalysator eignen sich Verbindungen der Übergangsmetalle aus der Platingruppe Ruthenium, Osmium, Rhodium, Iridium, Palladium und Platin, insbesondere Palladium und Ruthenium, die modifiziert mit ein- oder mehrzähnigen Organostickstoff- oder Organophosphorliganden oder in unmodifizierter Form eingesetzt werden können.

Das erhaltene Vinylisononanoat eignet sich als Comonomer in Polyvinylacetat, Polyvinylchlorid, Polystyrol oder Polyacrylsäureestern, das die Hydrolysebeständigkeit und Feuchtigkeitsaufnahme von Anstrichen vorteilhaft beeinflusst.

Aus der erfindungsgemäß hergestellten Isononansäure kann ebenfalls der entsprechende Glycidylester, beispielsweise durch Umsetzung mit Epichlorhydrin, nach an sich bekannten Verfahren hergestellt werden, der zur Modifizierung von Alkydharzen dienen kann (Weissermel, Arpe, Industrielle Organische Chemie, VCH Verlagsgesellschaft, 3. Auflage, 1988, Seite 152; US 6433217).

Die erfindungsgemäß hergestellte Isononansäure kann ebenfalls mit ein- oder mehrwertigen Alkoholen auf an sich bekannte Weise zu den entsprechenden Carbonsäureestern umgesetzt werden (Ullmanns Encyklopädie der technischen Chemie, 4. Auflage, 1976, Verlag Chemie, Band 11, Seiten 89-96), die in Schmierstoffzusammensetzungen, als Weichmacher für thermoplastische Polymere oder als Koaleszenzmittel in Dispersionsanstrichen verwendet werden können.

Ebenso kann die erfindungsgemäß hergestellte Isononansäure durch Umsetzung mit Halogenierungsmitteln wie Phosphorpentachlorid, Phosphoroxychlorid, Sulfurylchlorid oder Thionylchlorid in Isononansäurehalogenide derivatisiert werden, aus denen durch Umsetzung mit Isononansäure Isononansäureanyhdrid oder durch Umsetzung mit anderen Carbonsäuren gemischte Anhydride zugänglich sind. Auch die Umsetzung von Isononansäure mit Essigsäureanhydrid ergibt als Zwischenstufe das gemischte Anhydrid, das unter weiterer Säurezugabe und Essigsäureabspaltung in Isononansäureanhydrid oder in ein weiteres gemischtes Anhydrid umgewandelt werden kann (Ullmanns Encyklopädie der technischen Chemie, 4. Auflage, 1975, Verlag Chemie, Band 9, Seiten 145-146). Ausgehend von Isononansäurechlorid oder Isononansäureanhydrid können durch Umsetzung mit Ammoniak, primären oder sekundären Aminen die entsprechenden Isononansäureamide erhalten werden (Methoden der Organischen Chemie, Houben-Weyl, 4. Auflage, 1958, Georg Thieme Verlag, Stuttgart, Band XI/2, Seiten 10-14, 16-19).

In den folgenden Beispielen wird die Herstellung von Isononansäure ausgehend von 2-Ethylhexanol beschrieben.

### Beispiele

### I. Dehydratisierung von 2-Ethylhexanol

Zur Dehydratisierung wurde ein Quarzrohr mit einer Länge von 1,3 Meter und einem Durchmesser von 0,03 Meter verwendet, bei dem sich die beheizte Zone über 1,1 Meter erstreckte. Das Quarzrohr wurde mit 250 ml des sauren Katalysators Al 3996 der Firma BASF SE in Form von 3x3 Millimeter großen Tabletten bestückt. Das Totvolumen wurde mit Glasringen aufgefüllt. 2-Ethylhexanol wurde in einem vorgeschalteten Verdampfer verdampft und mit Hilfe eines Stickstoffstromes als Trägergas bei Normaldruck über die Katalysatorschüttung bei einer Temperatur von 350°C und mit einer Belastung von 0,5 Liter je Liter Katalysatorvolumen und Stunde gefahren. Das erhaltene Reaktionsgemisch wurde in einem nachgeschalteten Auffanggefäß kondensiert und die wässrige Phase wurde abgetrennt. Die angefallene organische Phase wies folgende gaschromatographisch ermittelte Zusammensetzung auf (FI.-%, gemäß DIN 51405):

| | |
|---|---|
| Vorlauf/C4-C7-Kohlenwasserstoffe | 0,3 |
| andere C8-Olefine | 9,6 |
| 2-Ethyl-1-hexen | 7,6 |
| cis-3-Methyl-3-hepten | 14,6 |
| trans-3-Methyl-3-hepten | 28,8 |
| cis-3-Methyl-2-hepten | 16,2 |
| trans-3-Methyl-2-hepten | 23,9 |
| n-Octene | 0,8 |
| Nachlauf | 0,1 |

### II. Hydroformylierung des gemäß Schritt I. erhaltenen Octens

Das nach Schritt I erhaltene rohe Octen wurde in Gegenwart von 5 ppm Rhodium, zugegeben in Form einer Lösung von Rhodium-2-ethylhexanoat in 2-Ethylhexanol und bezogen auf Octeneinsatz, bei einer Temperatur von 140°C und bei einem Synthesegasdruck von 19 MPa über einen Zeitraum von drei Stunden hydroformyliert. Die molare Zusammensetzung des Synthesegases betrug 1 Mol Wasserstoff zu 1 Mol Kohlenmonoxid. Das erhaltene rohe Hydroformylierungsprodukt wies folgende gaschromatographisch ermittelte Zusammensetzung (FI.-%, gemäß DIN 51405) auf:

| | |
|---|---|
| Vorlauf | 0,1 |
| C8-Kohlenwasserstoffe | 8,5 |
| Zwischenlauf | 0,2 |
| Isononanal | 88,1 |
| n-Nonanal | 1,4 |
| Nachlauf | 1,7 |

Die Ergebnisse weiterer Hydroformylierungsversuche mit einem über die Dehydratisierung von 2-Ethylhexanol erhaltenen Octen sind in der nachfolgenden Tabelle 1 zusammengestellt. Vor Einsatz wurde das rohe Octen an einer Claisenbrücke zur Nachlaufabtrennung bei einer Kopftemperatur von 119-122°C und bei Normaldruck destilliert. Die Einsatzoctene sowie die erhaltenen Reaktionsprodukte wurden gaschromatographisch analysiert (Angabe in FI.-%, gemäß DIN 51405).

**Tabelle 1: Hydroformylierung von Octenen, erhalten durch die 2-Ethylhexanoldehydratisierung**

| **Beispiel** | **IIa** | **IIb** |
|---|---|---|
| Einsatz Edukt | destilliert | destilliert |
| **GC**-**Analyse Edukt (%)** | | |
| Vorlauf/C4-C7-Kohlenwasserstoffe | 0,3 | 0,4 |
| andere C8-Olefine | 5,9 | 7,7 |
| 2-Ethyl-1-hexen | 9,3 | 9,2 |
| cis-3-Methyl-3-hepten | 15,2 | 15,0 |
| trans-3-Methyl-3-hepten | 27,4 | 27,1 |
| cis-3-Methyl-2-hepten | 16,1 | 15,6 |
| trans-3-Methyl-2-hepten | 25,2 | 24,7 |
| n-Octene | 0,5 | 0,2 |
| Nachlauf | 0,1 | 0,1 |

| **Versuchsbedingungen** | | |
|---|---|---|
| Rh-Konzentration [ppm], bezogen auf Octeneinsatz | 20 | 10 |
| Druck [MPa] | 19 | 27 |
| Temperatur [°C] | 140 | 140 |
| Reaktionszeit [h] | 2 | 2 |

| **GC-Analyse Produkt (%)** | | |
|---|---|---|
| Vorlauf | 0,1 | 0,1 |
| C8-Kohlenwasserstoffe | 2,5 | 1,1 |
| Zwischenlauf | 0,3 | 0,1 |
| iso-Nonanale | 90,8 | 94,7 |
| n-Nonanal | 2,0 | 1,4 |
| Nachlauf | 4,3 | 2,6 |

Die unter Verwendung von Triphenylphopshin als Komplexligand durchgeführten Hydroformylierungsversuche mit dem über die Dehydratisierung von 2-Ethylhexanol erhaltenen Octen sind in der nachfolgenden Tabelle 2 zusammengestellt. Es kam undestillierte Ware zum Einsatz. Die Einsatzoctene sowie die erhaltenen Reaktionsprodukte wurden gaschromatographisch analysiert (Angaben in FI.-%, gemäß DIN 51405).

**Tabelle 2: Hydroformylierung von Octenen, erhalten durch die 2-Ethylhexanoldehydratisierung, Zusatz von Triphenylphosphin**

| **Beispiel** | **IIc** | **IId** | **Iie** | **IIf** |
|---|---|---|---|---|
| Einsatz Edukt | undestilliert, roh | undestilliert, roh | undestilliert, roh | undestilliert, roh |
| **GC**-**Analyse Edukt (%)** | | | | |
| C4-C7 Kohlenwasserstoffe | 0,3 | 0,3 | 0,3 | 0,4 |
| Andere C8-Olefine | 19,1 | 19,1 | 19,1 | 11,6 |
| 2-Ethyl-1-hexen | 7,9 | 7,9 | 7,9 | 8,6 |
| 3-Methyl-3-hepten | 36,5 | 36,5 | 36,5 | 40,0 |
| 3-Methyl-2-hepten | 36,2 | 36,2 | 36,2 | 39,3 |
| Nachlauf | <0,01 | <0,01 | <0,01 | <0,1 |

| **Versuchsbedingungen** | | | | |
|---|---|---|---|---|
| Rh-Konzentration [ppm], bezogen auf Octeneinsatz | 10 | 10 | 10 | 10 |
| Äquivalente TPP | 3 | 50 | 100 | 3 |
| Druck [MPa] | 18 | 27 | 18 | 14 |
| Temperatur [°C] | 140 | 140 | 140 | 160 |
| Reaktionszeit [h] | 1 | 2 | 1 | 2 |

| **GC-Analyse Produkt (%)** | | | | |
|---|---|---|---|---|
| Vorlauf | 0,1 | 0,1 | 0,1 | 0,1 |
| C8 Kohlenwasserstoffe | 52,2 | 70,9 | 81,7 | 14,1 |
| Zwischenlauf | 0,8 | 0,1 | 0,1 | 1,9 |
| iso-Nonanale | 45,7 | 28,3 | 17,6 | 76,1 |
| n-Nonanal | 0,5 | 0,1 | 0,1 | 0,5 |
| Nachlauf | 0,7 | 0,4 | 0,4 | 7,3 |

### III. Oxidation des nach Schritt II. erhaltenen Isononanals zu Isononansäure

Aus dem nach Beispiel IIa erhaltenen Isononanal wurden zunächst Leichtsieder und unumgesetztes Olefin als Kopfprodukt an einer 24 Bödenkolonne bei 200 hPa, einer Sumpftemperatur von 120°C und einem Rücklaufverhältnis von 2:1 abgetrennt. Nach Leichtsiederabtrennung wurde die Sumpftemperatur auf 140-150°C angezogen und das Isononanal über Kopf abgezogen (Siedepunkt bei 100 hPa: 110-114°C), während Hochsieder im Destillationssumpf verblieben.

Das erhaltene Isononanal wies folgende gaschromatographisch ermittelte Zusammensetzung sowie folgende Kennzahlen auf und wurde für die nachfolgende Flüssigphasenoxidation eingesetzt.

**Tabelle 3: Gaschromatographische Analyse (FI-.%, gemäß DIN 51405) des Isononanals ausgehend von 2-Ethylhexanol**

| | |
|---|---|
| Vorlauf/C8-Kohlenwasserstoffe | 0,2 |
| Zwischenlauf | 0,4 |
| 2-Ethyl-4-methylhexanal | 10,8 |
| 2-Propyl-3-methylpentanal | 3,6 |
| 2,5-Dimethylheptanal | 21,9 |
| 2,3-Dimethylheptanal (Isomer) | 4,8 |
| 2,3-Dimethylheptanal (Isomer) + 2-Ethylheptanal | 8,4 |
| 2-Methyloctanal | 1,7 |
| 3-Ethylheptanal | 10,4 |
| 4-Methyloctanal | 20,6 |
| 4,5-Dimethylheptanal | 0,6 |
| 6-Methyloctanal | 11,0 |
| andere i-Nonanale | 1,8 |
| n-Nonanal | 0,9 |
| Nachlauf | 2,9 |

**Tabelle 4: Kennzahlen des Isononanals ausgehend von 2-Ethylhexanol**

| Kennzahl / Einheit | DIN/ASTM | Wert |
|---|---|---|
| V₂₀ (mm²/s) | ASTM D 445 | 1,536 |
| V₄₀ (mm²/s) | | 1,179 |
| Erstarrungspunkt (°C) | | -100 |
| d^{20/4} (g/cm³) | DIN 51757, Verf. D/ASTM D 4052 | 0,827 |
| d^{50/4} (g/cm³) | | 0,811 |
| n^{20/D} | DIN 51423-2/ ASTM D 1747 | 1,424 |
| CO-Zahl (mg KOH/g) | DIN 53173 | 339/349 |
| Flammpunkt (°C) | DIN EN ISO 2719 | 60 |
| Platin/Kobalt-Farbzahl Hazen | DIN EN ISO 6271/ ASTM D 1209 | 15 |

Die Flüssigphasenoxidation des Isononanals zu Isononansäure erfolgte ohne Lösungsmittelzusatz in einem Blasensäulenreaktor bei 50°C mit reinem Sauerstoff bei Normaldruck über einen Zeitraum von 6 Stunden. Dem Einsatzaldehyd wurde eine 50 Gew.-%ige wässrige Lösung von Kaliumhydroxid in einer solchen Menge zugesetzt, dass pro Mol Isononanal 50 mmol Kalium vorlagen.

Die erhaltene Rohsäure wurde anschließend an einer 4,5 Bödenkolonne bei einer Sumpftemperatur von 148 bis 159°C und bei einer Kopftemperatur von 136-139°C bei 20 hPa destilliert. Leichtsieder und unumgesetzter Aldehyd wurden als Vorlauffraktion abgetrennt und Schwersieder verblieben im Destillationsrückstand. Die Destillationsausbeute an Isononansäure betrug 84,7% mit einer gaschromatographisch ermittelten Reinheit von 98,8%.

Die erhaltene Isononansäure wies folgende nach DIN 51405 gaschromatographisch ermittelte Zusammensetzung (FI.-%) auf:

**Tabelle 5: Gaschromatographische Analyse der Isononansäure ausgehend von 2-Ethylhexanol (FI.-%, gemäß DIN 51405)**

| | |
|---|---|
| Vorlauf | 0,4 |
| 2-Ethyl-4-methylhexansäure | 9,3 |
| 2-Propyl-3-methylpentansäure | 3,0 |
| 2,5-Dimethylheptansäure + 2,3-Dimethylheptansäure (Isomer) | 25,7 |
| 2,3-Dimethylheptansäure (Isomer) | 8,4 |
| 3-Ethylheptansäure + 2-Ethylheptansäure | 12,9 |
| 2-Methyloctansäure | 0,8 |
| 4-Methyloctansäure | 20,9 |
| 6-Methyloctansäure | 12,3 |
| n-Nonansäure | 0,3 |
| andere i-Nonansäuren | 5,2 |
| Nachlauf | 0,8 |

Die für die Isononansäure ermittelten Kennzahlen sind in der Tabelle 6 zusammengestellt.

**Tabelle 6: Kennzahlen der Isononansäure ausgehend von 2-Ethylhexanol**

| Kennzahl / Einheit | DIN/ASTM | Wert |
|---|---|---|
| V₂₀ (mm²/s) | ASTM D 445 | 10,68-11,18 |
| V₄₀ (mm²/s) | | 5,83-5,88 |
| V₅₀ (mm²/s) | | 4,50 |
| d^{20/4} (g/cm³) | DIN 51757, Verf. D/ | 0,906-0,907 |
| d^{40/4} (g/cm³) | ASTM D 4052 | 0,891 |
| d^{50/4} (g/cm³) | | 0,883-0,884 |
| n^{20/D} | DIN 51 423-2/ ASTM D 1747 | 1,432-1,433 |
| Erstarrungspunkt (°C) | | -81 |
| Siedepunkt (°C) bei 1013 hPa | DIN 53171 / ASTM D 1078 | 241-242 |
| Säurezahl (mg KOH/g) | DIN EN ISO 2114/ ASTM D 1613 | 351 |
| Flammpunkt (°C) | DIN EN ISO 2719 | 129 |
| Platin/Kobalt-Farbzahl Hazen | DIN EN ISO 6271/ ASTM D 1209 | 7 |

## Patentansprüche

1. Verfahren zur Herstellung von Isononansäure ausgehend von 2-Ethylhexanol, **dadurch gekennzeichnet, dass** man
(a) 2-Ethylhexanol in Gegenwart eines Katalysators zu einem Octengemisch dehydratisiert;
(b) das nach Schritt a) erhaltene Octengemisch in Gegenwart einer Übergangsmetallverbindung der Gruppe VIII des Periodensystems der Elemente mit Kohlenmonoxid und Wasserstoff zu Isononanal umsetzt; und
(c) das nach Schritt b) erhaltene Isononanal zu Isononansäure oxidiert, wobei Isononansäure als Gemisch strukturverzweigter C9-Monocarbonsäuren und Isononanal als Gemisch isomerer Isononanale definiert sind.

2. Verfahren gemäß Anspruch 1, **dadurch gekennzeichnet, dass** man in Schritt a) als Katalysator Aluminiumoxid, Nickel niedergeschlagen auf Aluminiumoxid, oder Phosphorsäure niedergeschlagen auf Siliziumdioxid oder Aluminiumoxid verwendet.

3. Verfahren gemäß Anspruch 1 oder 2, **dadurch gekennzeichnet, dass** man in Schritt a) 2-Ethylhexanol in der Gasphase dehydratisiert.

4. Verfahren gemäß einem oder mehreren der Ansprüche 1 bis 3, **dadurch gekennzeichnet, dass** man in Schritt b) als Übergangsmetallverbindung der Gruppe VIII des Periodensystems der Elemente eine Kobalt- oder Rhodiumverbindung verwendet.

5. Verfahren gemäß einem oder mehreren der Ansprüche 1 bis 4, **dadurch gekennzeichnet, dass** die Umsetzung gemäß Schritt b) in Abwesenheit von komplexbildenden Organoelementverbindungen durchgeführt wird.

6. Verfahren gemäß einem oder mehreren der Ansprüche 1 bis 5, **dadurch gekennzeichnet, dass** man das nach Schritt b) erhaltene Isononanal destilliert.

7. Verfahren gemäß einem oder mehreren der Ansprüche 1 bis 6, **dadurch gekennzeichnet, dass** die Oxidation in Schritt c) in Anwesenheit von Alkali- oder Erdalkalimetallcarboxylaten erfolgt.

8. Verfahren gemäß Anspruch 7, **dadurch gekennzeichnet, dass** man als Alkali- oder Erdalkalimetallcarboxylat Lithium-, Kalium-, Natrium-, Calcium- oder Bariumisononanoat verwendet.

9. Verfahren gemäß einem oder mehreren der Ansprüche 1 bis 8, **dadurch gekennzeichnet, dass** man in Schritt c) Isononanal in der Flüssigphase oxidiert.

10. Verfahren gemäß einem oder mehreren der Ansprüche 1 bis 9, **dadurch gekennzeichnet, dass** man in Schritt c) Isononanal mit Sauerstoff oder sauerstoffenthaltenden Gasen zu Isononansäure oxidiert.

11. Verfahren zur Herstellung von Vinylisononanoat, **dadurch gekennzeichnet, dass** man die Isononansäure gemäß den Ansprüchen 1 bis 10 herstellt und mit Acetylen auf an sich bekannte Weise umsetzt.

12. Verfahren zur Herstellung von Vinylisononanoat, **dadurch gekennzeichnet, dass** man die Isononansäure gemäß den Ansprüchen 1 bis 10 herstellt und mit Vinylacetat oder Vinylpropionat auf an sich bekannte Weise umsetzt.

13. Verfahren zur Herstellung von Carbonsäureestern, **dadurch gekennzeichnet, dass** man die Isononansäure gemäß den Ansprüchen 1 bis 10 herstellt und mit ein- oder mehrwertigen Alkoholen auf an sich bekannte Weise verestert.

14. Verfahren zur Herstellung von Isononansäurehalogeniden, **dadurch gekennzeichnet, dass** man die Isononansäure gemäß den Ansprüchen 1 bis 10 herstellt und mit Halogenierungsmitteln auf an sich bekannte Weise umsetzt.

15. Verfahren zur Herstellung von Isononansäureanhydriden, **dadurch gekennzeichnet, dass** man die Isononansäure gemäß den Ansprüchen 1 bis 10 herstellt und mit Halogenierungsmitteln und anschließend mit Carbonsäuren auf an sich bekannte Weise umsetzt.

16. Verfahren zur Herstellung von Isononansäureamiden, **dadurch gekennzeichnet, dass** man die Isononansäure gemäß den Ansprüchen 1 bis 10 herstellt und in Isononansäurechlorid oder Isononansäureanhydrid überführt und anschließend mit Ammoniak, primären oder sekundären Aminen auf an sich bekannte Weise umsetzt.

17. Verfahren zur Herstellung des Glycidylesters der Isononansäure, **dadurch gekennzeichnet, dass** man die Isononansäure gemäß den Ansprüchen 1 bis 10 herstellt und auf an sich bekannte Weise zu dem Glycidylester umsetzt.

## Claims

1. Process for preparing isononanoic acid proceeding from 2-ethylhexanol, **characterized in that**
(a) 2-ethylhexanol is dehydrated to an octene mixture in the presence of a catalyst;
(b) the octene mixture obtained in step a) is reacted in the presence of a transition metal compound of group VIII of the periodic table of the elements with carbon monoxide and hydrogen to give isononanal; and
(c) the isononanal obtained in step b) is oxidized to isononanoic acid, where isononanoic acid is defined as a mixture of structurally branched C9 monocarboxylic acids and isonanal as a mixture of isomeric isononanols.

2. Process according to Claim 1, **characterized in that** the catalyst used in step a) is alumina, nickel precipitated on alumina, or phosphoric acid precipitated on silica or alumina.

3. Process according to Claim 1 or 2, **characterized in that** 2-ethylhexanol is dehydrated in the gas phase in step a).

4. Process according to one or more of Claims 1 to 3, **characterized in that** the transition metal compound of group VIII of the periodic table of the elements used in step b) is a cobalt or rhodium compound.

5. Process according to one or more of Claims 1 to 4, **characterized in that** the reaction in step b) is performed in the absence of complex-forming organoelemental compounds.

6. Process according to one or more of Claims 1 to 5, **characterized in that** the isononanal obtained in step b) is distilled.

7. Process according to one or more of Claims 1 to 6, **characterized in that** the oxidation in step c) is effected in the presence of alkali metal or alkaline earth metal carboxylates.

8. Process according to Claim 7, **characterized in that** the alkali metal or alkaline earth metal carboxylate used is lithium isononanoate, potassium isononanoate, sodium isononanoate, calcium isononanoate or barium isononanoate.

9. Process according to one or more of Claims 1 to 8, **characterized in that** isononanal is oxidized in the liquid phase in step c).

10. Process according to one or more of Claims 1 to 9, **characterized in that** isononanal is oxidized in step c) with oxygen or oxygen-containing gases to isononanoic acid.

11. Process for preparing vinyl isononanoate, **characterized in that** the isononanoic acid is prepared according to Claims 1 to 10 and is reacted with acetylene in a manner known per se.

12. Process for preparing vinyl isononanoate, **characterized in that** the isononanoic acid and according to Claims 1 to 10 is reacted with vinyl acetate or vinyl propionate in a manner known per se.

13. Process for preparing carboxylic esters, **characterized in that** the isononanoic acid is prepared according to Claims 1 to 10 and is esterified with mono- or polyhydric alcohols in a manner known per se.

14. Process for preparing isononanoyl halides, **characterized in that** the isononanoic acid is prepared according to Claims 1 to 10 and is reacted with halogenating agents in a manner known per se.

15. Process for preparing isononanoic anhydrides, **characterized in that** the isononanoic acid is prepared according to Claims 1 to 10 and is reacted with halogenating agents and then with carboxylic acids in a manner known per se.

16. Process for preparing isononanamides, **characterized in that** the isononanoic acid is prepared according to Claims 1 to 10 and is converted to isononanoyl chloride or isononanoic anhydride and then reacted with ammonia, primary or secondary amines in a manner known per se.

17. Process for preparing the glycidyl ester of isononanoic acid, **characterized in that** the isononanoic acid is prepared according to Claims 1 to 10 and is converted to the glycidyl ester in a manner known per se.

## Revendications

1. Procédé de fabrication d'acide isononanoïque à partir de 2-éthylhexanol, **caractérisé en ce que**
(a) du 2-éthylhexanol est déshydraté en présence d'un catalyseur pour former un mélange d'octènes ;
(b) le mélange d'octènes obtenu à l'étape a) est mis en réaction en présence d'un composé de métal de transition du groupe VIII du tableau périodique des éléments avec du monoxyde de carbone et de l'hydrogène pour former de l'isononanal ; et
(c) l'isononanal obtenu à l'étape b) est oxydé en acide isononanoïque,
l'acide isononanoïque étant défini comme un mélange d'acides monocarboxyliques en C9 de structure ramifiée et l'isononanal comme un mélange d'isononanals isomères.

2. Procédé selon la revendication 1, **caractérisé en ce que** de l'oxyde d'aluminium, du nickel précipité sur de l'oxyde d'aluminium ou de l'acide phosphorique précipité sur du dioxyde de silicium ou de l'oxyde d'aluminium est utilisé en tant que catalyseur à l'étape a).

3. Procédé selon la revendication 1 ou 2, **caractérisé en ce que** le 2-éthylhexanol est déshydraté dans la phase gazeuse à l'étape a).

4. Procédé selon une ou plusieurs des revendications 1 à 3, **caractérisé en ce qu'**un composé de cobalt ou de rhodium est utilisé en tant que composé de métal de transition du groupe VIII du tableau périodique des éléments à l'étape b).

5. Procédé selon une ou plusieurs des revendications 1 à 4, **caractérisé en ce que** la réaction selon l'étape b) est réalisée en l'absence de composés d'organo-éléments complexants.

6. Procédé selon une ou plusieurs des revendications 1 à 5, **caractérisé en ce que** l'isononanal obtenu à l'étape b) est distillé.

7. Procédé selon une ou plusieurs des revendications 1 à 6, **caractérisé en ce que** l'oxydation à l'étape c) a lieu en présence de carboxylates de métaux alcalins ou alcalino-terreux.

8. Procédé selon la revendication 7, **caractérisé en ce que** de l'isononanoate de lithium, potassium, sodium, calcium ou baryum est utilisé en tant que carboxylate de métal alcalin ou alcalino-terreux.

9. Procédé selon une ou plusieurs des revendications 1 à 8, **caractérisé en ce que** l'isononanal est oxydé dans la phase liquide à l'étape c).

10. Procédé selon une ou plusieurs des revendications 1 à 9, **caractérisé en ce que** l'isononanal est oxydé avec de l'oxygène ou des gaz contenant de l'oxygène pour former l'acide isononanoïque à l'étape c).

11. Procédé de fabrication d'isononanoate de vinyle, **caractérisé en ce que** l'acide isononanoïque est fabriqué selon les revendications 1 à 10 et mis en réaction avec de l'acétylène d'une manière connue en soi.

12. Procédé de fabrication d'isononanoate de vinyle, **caractérisé en ce que** l'acide isononanoïque est fabriqué selon les revendications 1 à 10 et mis en réaction avec de l'acétate de vinyle ou du propionate de vinyle d'une manière connue en soi.

13. Procédé de fabrication d'esters d'acides carboxyliques, **caractérisé en ce que** l'acide isononanoïque est fabriqué selon les revendications 1 à 10 et estérifié avec des alcools mono- ou polyvalents d'une manière connue en soi.

14. Procédé de fabrication d'halogénures de l'acide isononanoïque, **caractérisé en ce que** l'acide isononanoïque est fabriqué selon les revendications 1 à 10 et mis en réaction avec des agents d'halogénation d'une manière connue en soi.

15. Procédé de fabrication d'anhydrides de l'acide isononanoïque, **caractérisé en ce que** l'acide isononanoïque est fabriqué selon les revendications 1 à 10 et mis en réaction avec des agents d'halogénation, puis avec des acides carboxyliques d'une manière connue en soi.

16. Procédé de fabrication d'amides de l'acide isononanoïque, **caractérisé en ce que** l'acide isononanoïque est fabriqué selon les revendications 1 à 10 et transformé en chlorure d'acide isononanoïque ou anhydride d'acide isononanoïque, puis mis en réaction avec de l'ammoniac, des amines primaires ou secondaires d'une manière connue en soi.

17. Procédé de fabrication de l'ester glycidylique de l'acide isononanoïque, **caractérisé en ce que** l'acide isononanoïque est fabriqué selon les revendications 1 à 10 et transformé en l'ester glycidylique d'une manière connue en soi.
